# EUROPÄISCHE PATENTSCHRIFT

(11) **EP 1 354 591 B1**
(45) Veröffentlichungstag und Bekanntmachung des Hinweises auf die Patenterteilung: **15.06.2005**
(21) Anmeldenummer: 03008732.4
(22) Anmeldetag: 16.04.2003
(51) Int. Cl.: A61K 35/02, A61K 7/06, A61K 7/48, A61P 17/00, A61P 31/02

(54) **Topische Zusammensetzung enthaltend Rauchagglomerate**
Topical composition containing agglomerated particles from smoke
Composition topique contenant des particules agglomérées de la fumée

(30) Priorität: 17.04.2002 DE 10217076
(43) Veröffentlichungstag der Anmeldung: 22.10.2003
(73) Patentinhaber: MVB GmbH, 22767 Hamburg (DE)
(72) Erfinder: Zavrakis, Wassili, 22880 Wedel (DE)
(74) Vertreter: UEXKÜLL & STOLBERG

(56) Entgegenhaltungen:
- DE-C- 4 320 523
- MASON J ET AL: "Topical preparations for the treatment of psoriasis: a systematic review." THE BRITISH JOURNAL OF DERMATOLOGY. ENGLAND MAR 2002, Bd. 146, Nr. 3, März 2002 (2002-03), Seiten 351-364, XP002244836 ISSN: 0007-0963
- JUNG T: "New treatments for atopic dermatitis." CLINICAL AND EXPERIMENTAL ALLERGY, Bd. 32, Nr. 3, März 2002 (2002-03), Seiten 347-354, XP002244837 March, 2002 ISSN: 0954-7894
- DATABASE MEDLINE [Online] US NATIONAL LIBRARY OF MEDICINE (NLM), BETHESDA, MD, US; 2000 HOARE C ET AL: "Systematic review of treatments for atopic eczema." Database accession no. NLM11134919 XP002244838 & HEALTH TECHNOLOGY ASSESSMENT (WINCHESTER, ENGLAND) ENGLAND 2000, Bd. 4, Nr. 37, 2000, Seiten 1-191, ISSN: 1366-5278

## Beschreibung

Die Erfindung betrifft eine topische Zusammensetzung, die Rauchagglomerate enthält, die durch Verglimmen von gewöhnlichem bedruckten Zeitungspapier erhältlich sind sowie dessen Verwendung zur Behandlung von Hauterkrankungen, insbesondere Neurodermitis und Schuppenflechte, Juckreiz und Ekzemen sowie als Desinfektionsmittel und Haarwaschmittel.

Neurodermitis (atopische Dermatitis) ist eine dauerhaft juckende Entzündung der Haut. Im Allgemeinen haben Menschen mit Neurodermitis eine trockene Haut, Juckreiz und eine Neigung zu Infektionskrankheiten der Haut. Schuppenflechte (Psoriasis) ist eine chronische, entzündliche und immer wiederkehrende Hautkrankheit mit erhöhter Schuppenbildung.

In der Medizin gibt es zahlreiche Mittel, die zur äußerlichen Behandlung solcher Hauterkrankungen eingesetzt werden. Dazu gehören u.a. Steinkohlenteer, Kortison, Vitamin-A- und Vitamin-D-Derivate.

Steinkohlenteer kann in den unterschiedlichsten Darreichungsformen auf die Haut appliziert werden, z.B. als Fettcreme und Lotion (z.B. Poloris®), Gel (Basiter®) oder als Lösung (z.B. Tarmed®). Der durch trockene Destillation von Steinkohle gewonnene Steinkohlenteer ist ein komplexes Gemisch unterschiedlichster Substanzen und enthält u.a. Naphthalin, Phenol und Phenolderivate, Anthracen sowie Benzpyrene. Wegen relativ hoher Mengen an kanzerogener Stoffe wird empfohlen, Steinkohlenteerpräparate nur in begründeten und vom Arzt ständig kontrollierten Fällen und nur für kurze Zeit therapeutisch einzusetzen. Auf Hautstellen, wo verstärkt schädliche Substanzen aufgenommen werden (z.B. im Genitalbereich), sowie während der Schwangerschaft oder der Stillzeit sollte Steinkohlenteer generell nicht angewendet werden. Der Einsatz von Steinkohlenteer in Kosmetika ist aufgrund der krebserregenden Inhaltsstoffe bereits verboten worden.

Auch Präparate auf Basis von Kortison, einem Hormon der Nebennierenrinde, sind problematisch, weil bei längerer Anwendung ein Dünnerwerden der behandelten Haut (Hautatrophie) auftreten kann. Eine Applikation von Kortisonsalben auf größeren Hautflächen und an bestimmten Stellen (z.B. Genitalbereich) wird daher generell nicht empfohlen.

Zwar stellen Vitamin-A- und Vitamin-D-Abkömmlinge (z.B. Zorac®, Silkis®) eine Alternative zu Kortisonpräparaten dar, jedoch sollten sie wegen möglicher Reizerscheinungen der Haut beispielsweise nicht im Gesichtsbereich angewendet werden. Außerdem sind Vitamin-A-Abkömmlinge teratogen und kommen daher für eine therapeutische Behandlung während der Schwangerschaft nicht in Betracht.

Daher liegt der vorliegenden Erfindung die Aufgabe zugrunde, ein Mittel zur Verfügung zu stellen, die sich zur Behandlung von Hauterkrankungen wie Neurodermitis und Schuppenflechte eignet, jedoch die Nachteile der aus dem Stand der Technik bekannten Präparate nicht aufweist.

Diese Aufgabe wird durch eine topische Zusammensetzung gelöst, die Rauchagglomerate enthält, die erhältlich sind durch Verglimmen von gewöhnlichem, bedrucktem Zeitungspapier.

Gegenstand der Erfindung ist auch die Verwendung der topischen Zusammensetzung zur Behandlung von Hauterkrankungen, insbesondere Neurodermitis und Schuppenflechte, Juckreiz oder Ekzemen.

Gegenstand der Erfindung ist ferner die Verwendung der topischen Zusammensetzung als Desinfektionsmittel und/oder Haarwaschmittel.

Die im Folgenden angegebenen Gewichtsprozentangaben beziehen sich, soweit nicht anders angegeben, auf das Gewicht der gesamten topischen Zusammensetzung.

Unter einer topischen Zusammensetzung wird allgemein eine Zusammensetzung verstanden, die eine Applikation auf der Haut ermöglicht und mindestens einen dermatologischen Zweck erfüllen soll.

Die erfindungsgemäße topische Zusammensetzung enthält Rauchagglomerate. Im Allgemeinen entstehen Rauchagglomerate dadurch, dass der durch unvollständige Verbrennung von organischen Materialien, z.B. Zeitungspapier, entstehende Rauch, der in einem Gas, z.B. Luft, feinteilig dispergierte Feststoffpartikel darstellt, sich bei hohen Rauchkonzentrationen zu Teilchen mit größeren Durchmessern (Rauchagglomeraten) zusammenschließt.

Die Rauchagglomerate in der erfindungsgemäßen topischen Zusammensetzung sind erhältlich durch Verglimmen von gewöhnlichem, bedrucktem Zeitungspapier.

Unter Verglimmen wird verstanden, dass eine langsame Verbrennung des Zeitungspapiers unter verminderter Sauerstoffzufuhr erfolgt. Dies wird im vorliegenden Fall dadurch erreicht, dass Zeitungspapier zu einer konisch verlaufenden Tüte (Kegel) geformt und mit der Spitze nach oben auf einer tellerähnlichen Unterlage gestellt wird. Die Spitze des Kegels wird angezündet. Es entsteht dabei eine kleine offene Flamme entlang des oberen Papierrandes mit gelblicher Färbung, wobei kaum Glut entsteht.

Bei dem verwendeten Zeitungspapier handelt es sich um gewöhnliches, mit Druckerschwärze bedrucktes Zeitungspapier, wie es von üblichen Tageszeitungen bekannt ist. Alternativ kann auch unbedrucktes Zeitungspapier verwendet werden.

In einer bevorzugten Ausführungsform sind die Rauchagglomerate erhältlich durch
a) Formen von gewöhnlichem bedrucktem Zeitungspapier zu einem Kegel,
b) Anordnen des Kegels mit seiner Grundfläche auf einer feuerfesten Unterlage,
c) Entzünden des Kegels im Bereich seiner Spitze,
d) Verglimmen des Kegels und
e) Abscheiden des beim Verglimmen entstehenden Rauchs auf der feuerfesten Unterlage als Rauchagglomerate.

In Schritt b) kommen als feuerfeste Unterlage beispielsweise übliche Porzellanteller, zu Platten geformte Ton- oder Schamotterzeugnisse und dergleichen in Betracht. Besonders bevorzugt sind Glasunterlagen.

Nach Entzünden des Kegels im Bereich seiner Spitze (Schritt c)) verglimmt das Papier langsam in Richtung der feuerfesten Unterlage. Der dabei entstehende Rauch entweicht in Richtung der feuerfesten Unterlage und schlägt sich darauf in Form von Rauchagglomeraten nieder. Es wird vermutet, dass durch die erhöhte Rauchkonzentration innerhalb des Kegels eine Agglomeration der feinteilig dispergierten Rauchpartikel zu größeren Teilchen (Rauchagglomeraten) stattfindet, die sich auf der feuerfesten Unterlage abscheiden. Dieser Vorgang setzt sich fort, bis etwa die Hälfte des Kegels verglimmt ist. Danach findet keine Abscheidung mehr statt. Der Rauch kann nunmehr nach oben entweichen und die Flamme wird durch offensichtliche Sauerstoffzufuhr größer.

Die Rauchagglomerate können in einer beliebigen wirksamen Menge in der erfindungsgemäßen topischen Zusammensetzung enthalten sein. Deren Gehalt richtet sich nach unterschiedlichen Faktoren, z.B. dem Anwendungszweck, der Behandlungszeit oder der Form, in der die topische Zusammensetzung vorliegt. Somit kann die topische Zusammensetzung durch Variation des Gehalts an Rauchagglomeraten an das jeweilige Anwendungsgebiet und therapeutische Erfordernisse angepasst werden.

Vorzugsweise liegt die erfindungsgemäße topische Zusammensetzung als halbfeste Darreichungsform oder als flüssige Zubereitung vor. Bevorzugte halbfeste Darreichungsformen sind ausgewählt aus Salben, Cremes, Gelen und Pasten. Unter den flüssigen Zubereitungen sind Lösungen und/oder Suspensionen bevorzugt. Es ist auch möglich, die erfindungsgemäße topische Zusammensetzung in wässrige Tensidzubereitungen, z.B. Schampoos oder Duschgels, einzuarbeiten, womit Kopfhaut und andere Hautstellen gereinigt werden können.

Die erfindungsgemäße topische Zusammensetzung kann auch lediglich aus den Rauchagglomeraten bestehen. Da die Rauchagglomerate nach dem Verglimmen des Zeitungspapiers als zähflüssiges Produkt anfallen, das an der Luft relativ schnell eintrocknet und einen als unangenehm empfundenen Geruch aufweisen kann, ist eine solche Zusammensetzung jedoch weniger bevorzugt.

Wenn die topische Zusammensetzung in einer halbfesten Darreichungsform, z.B. als Salbe, vorliegt, enthält sie die Rauchagglomerate, vorzugsweise in einer Menge von 1 bis 20 Gew.-%, bevorzugter 5 bis 15 Gew.-%. Typische Mengen an Rauchagglomeraten in einer flüssigen Zubereitung liegen im Bereich von 1 bis 20 Gew.-%, bevorzugter 5 bis 15 Gew.-%. Bei Konzentrationen über 5 Gew.-% tritt sowohl bei Salben als auch bei flüssigen Zubereitungen eine deutliche Verbesserung der Wirkung ein.

Abgesehen von den Rauchagglomeraten enthalten die erfindungsgemäßen halbfesten Darreichungsformen sowie flüssigen Zubereitungen Komponenten, wie sie üblicherweise für solche Formulierungstypen eingesetzt werden. Beispielsweise enthalten erfindungsgemäße Salben Fette, Öle, Wachse und/oder Fettkörper, Wasser sowie mindestens einen Emulgator. Es ist auch möglich, dass die erfindungsgemäße Salbe ein oder mehrere der folgenden Zusatzstoffe enthält: Antioxidantien, UV-Absorber, anorganische Pigmente.

Auch die Verfahren zur Herstellung der erfindungsgemäßen halbfesten Darreichungsformen und flüssigen Zubereitungen sind solche, wie sie in der pharmazeutischen Technologie für solche Formulierungstypen üblich sind. Darüber hinaus ist es für Fachleute selbstverständlich, dass das jeweilige Herstellungsverfahren hinsichtlich der Eigenschaften und der Menge des Wirkstoffs, der physiologischen und anatomischen Verhältnisse am Applikationsort, der therapeutischen Anforderungen und der maschinellen Gegebenheiten optimiert werden kann.

In überraschender Weise hat sich gezeigt, dass sich die erfindungsgemäße topische Zusammensetzung mit einem Gehalt an den oben beschriebenen Rauchagglomeraten zur wirksamen Behandlung von chronischen entzündlichen Hauterkrankungen wie Neurodermitis und Schuppenflechte eignet. Außerdem war es vorteilhaft, dass Ekzemen (auf verschiedensten Ursachen beruhende Hautausschläge) entgegengewirkt werden konnte. Verschiedene Formen, in denen solche Hautausschläge auftreten, u.A. Knötchen-, Pustel-, Bläschen-, Schuppenbildung, Schwellung und Rötung, Juckreiz und Nesseln gingen bei Anwendung der erfindungsgemäßen topischen Zusammensetzung teilweise oder sogar vollständig zurück. Darüber hinaus konnten Folgen allergischer Reaktionen auf Haustiere (z.B. Katzen und Hunde) wirksam bekämpft werden.

In besonders vorteilhafter Weise kann die Zusammensetzung in Kosmetika eingesetzt werden, da sie im Gegensatz zu z.B. Steinkohlenteer keine krebserzeugenden Verbindungen, z.B. 4-Methoxyphenol, enthält.

Ferner verfügt die erfindungsgemäße topische Zusammensetzung über desinfizierende Eigenschaften, so dass auch ihr Einsatz als Desinfektionsmittel in Betracht kommt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zusammensetzungen.

### Beispiele

Ein Blatt übliches weißes bedrucktes Zeitungspapier wurde zu einem Kegel zusammengerollt und mit der Spitze nach oben auf einen tellerähnlichen Untersatz gestellt. Die Spitze des Kegels wurde angezündet, woraufhin das Papier langsam nach unten verglimmte. Der hierbei entstehende Rauch entwich nach unten und schlug sich als Rauchagglomerate auf dem Untersatz nieder. Dieser Niederschlag wurde abgeschabt und in einen luftdichten Behälter überführt und darin aufbewahrt. Der Vorgang wurde solange wiederholt, bis genügend topische Zusammensetzung für Behandlungszwecke zusammengekommen waren.

Mit dieser topischen Zusammensetzung, die lediglich aus den Rauchagglomeraten bestand, wurden die folgenden Testpersonen, die unterschiedliche Erkrankungen aufwiesen, behandelt.

Testperson 1 (männlich, 45 Jahre) hatte Schuppenflechte seit seinem 12. Lebensjahr. Durch dreimalige Behandlung mit der topischen Zusammensetzung erfolgte eine vollständige Heilung, ohne dass weitere Beschwerden auftraten.

Testperson 2 (weiblich, 43 Jahre) hatte über den gesamten Körper verteilt durch Katzenflöhe verursachte Hautflechten und leidete an starkem Juckreiz. Die entsprechenden Hautstellen wurden in einer dicken Schicht mit der topischen Zusammensetzung eingerieben. Nach etwa 1 Tag ging die Hautflechtenbildung sichtbar zurück und der Juckreiz nahm spürbar ab. Nach 5 Behandlungen mit der topischen Zusammensetzung innerhalb von 2 Wochen heilten alle vormals mit Hautflechten befallenden Stellen ab und der Juckreiz war vollständig beseitigt. Seitdem traten keine weiteren Beschwerden auf.

Testperson 3 (weiblich, 20 Jahre) wies dieselben Symptome wie Testperson 2 auf. Die bei Testperson 2 vorgenommene Behandlung verlief hier ebenfalls positiv. Bei der Testperson 3 trat später eine durch Reinigungsmittel ausgelöste starkjuckende allergische Hautreaktion auf, die mit der topischen Zusammensetzung behandelt wurde. Nach etwa 4 Behandlungen innerhalb 1 Woche war eine vollständige Heilung eingetreten.

Testperson 4 (männlich, 13 Jahre) wies wie die Testpersonen 2 und 3 Hautflechten auf. Die bei letzteren Testpersonen vorgenommene Behandlung führte hier ebenfalls zu einem positiven Ergebnis.

Testperson 5 (weiblich, 21 Jahre) hatte seit Kindheit Neurodermitis. Sie wurde über einen Zeitraum von 6 Wochen mehrfach mit der topischen Zusammensetzung behandelt. Danach waren etwa 85 bis 90 % der erkrankten Flächen abgeheilt.

Testperson 6 (weiblich, 40 Jahre) wies nesselnde Schuppenflechte am ganzen Körper auf. Bei einer Behandlung mit der topischen Zusammensetzung am Arm gingen die erkrankten Hautstellen um etwa 50 % zurück (die Flechte wurde kleiner und vergrößerte sich nicht mehr).

Testperson 7 (männlich, 30 Jahre) wies Schuppenflechte seit der Kindheit am ganzen Körper auf. Dreimalige Behandlung mit der topischen Zusammensetzung über einen Zeitraum von 6 Wochen führte zur vollständigen Heilung. Es traten keine weiteren Beschwerden mehr auf.

Testperson 8 (weiblich, 43 Jahre) wies Neurodermitis an einer Hand mit Rissbildung auf. Eine zweimalige Anwendung der topischen Zusammensetzung in einer Woche führte anschließend zur vollständigen Abheilung der erkrankten Stellen.

## Patentansprüche

1. Topische Zusammensetzung, enthaltend Rauchagglomerate, die erhältlich sind durch Verglimmen von gewöhnlichem, bedrucktem Zeitungspapier.

2. Topische Zusammensetzung nach Anspruch 1, **dadurch gekennzeichnet, dass** sie als halbfeste Darreichungsform oder als flüssige Zubereitung vorliegt.

3. Topische Zusammensetzung nach Anspruch 2, **dadurch gekennzeichnet, dass** die halbfeste Darreichungsform ausgewählt ist aus Salben, Cremes, Gels und Pasten.

4. Topische Zusammensetzung nach Anspruch 2, **dadurch gekennzeichnet, dass** die flüssige Zubereitung eine Lösung oder Suspension ist.

5. Verwendung einer topischen Zusammensetzung gemäß einem der Ansprüche 1 bis 4 zur Herstellung eines Medikaments zur Behandlung von Hauterkrankungen, Juckreiz oder Ekzemen.

6. Verwendung nach Anspruch 5, **dadurch gekennzeichnet, dass** es sich bei den Hauterkrankungen um Neurodermitis und/oder Schuppenflechte (Psoriasis) handelt.

7. Verwendung einer topischen Zusammensetzung gemäß einem der Ansprüche 1 bis 4 zur Herstellung eines Desinfektionsmittels.

8. Verwendung einer topischen Zusammensetzung gemäß einem der Ansprüche 1 bis 4 als Haarwaschmittel.

## Claims

1. A topical composition, containing smoke agglomerates, which can be obtained by ordinary, printed newspaper smouldering away.

2. A topical composition according to Claim 1,
**characterised in that** it exists as a semi-solid form of administration or as a liquid preparation.

3. A topical composition according to Claim 2,
**characterised in that** the semi-sold form of administration is selected from ointments, creams, gels and pastes.

4. A topical composition according to Claim 2,
**characterised in that** the liquid preparation is a solution or a suspension.

5. Use of a topical composition in accordance with one of Claims 1 to 4 for the manufacture of a drug for the treatment of skin diseases, pruritis or eczema.

6. Use according to Claim 5,
**characterised in that** the skin diseases are neurodermatitis and/or psoriasis.

7. Use of a topical composition according to one of Claims 1 to 4 for the manufacture of a disinfectant.

8. Use of a topical composition according to one of Claims 1 to 4 as a shampoo.

## Revendications

1. Composition topique contenant des agglomérats de fumée qui peuvent être obtenus par consumation de papier journal imprimé courant.

2. Composition topique selon la revendication 1, **caractérisée en ce qu'**elle est présente sous une forme d'administration semi-solide ou sous forme d'une préparation liquide.

3. Composition topique selon la revendication 2, **caractérisée en ce que** la forme d'administration semi-solide est choisie parmi les pommades, les crèmes, les gels et les pâtes.

4. Composition topique selon la revendication 2, **caractérisée en ce que** la préparation liquide est une solution ou une suspension.

5. Utilisation d'une composition topique selon l'une des revendications 1 à 4 pour la production d'un médicament pour le traitement des maladies cutanées, du prurit ou de l'eczéma.

6. Utilisation selon la revendication 5, **caractérisée en ce que** les maladies cutanées sont la neurodermatite et/ou le psoriasis.

7. Utilisation d'une composition topique selon l'une des revendications 1 à 4 pour la production d'un désinfectant.

8. Utilisation d'une préparation topique selon l'une des revendications 1 à 4 comme shampoing.
